# EUROPEAN PATENT SPECIFICATION

(11) **EP 1 830 827 B1**
(45) Date of publication and mention of the grant of the patent: **02.03.2011**
(21) Application number: 05811366.3
(22) Date of filing: 01.12.2005
(51) Int. Cl.: A61K 31/02, A61P 35/00

(54) **PERFLUOROCARBON LIQUIDS TO REMOVE CARCINOGENS**
PERFLUORKOHLENSTOFF-FLÜSSIGKEITEN ZUR ENTFERNUNG VON KARZINOGENEN
CARBONES PERFLUORES LIQUIDES POUR ELIMINER LES CARCINOGENES

(30) Priority: 02.12.2004 GB 0426489
(43) Date of publication of application: 12.09.2007
(73) Proprietor: Matsumoto, Kaizen Robert, 18 Chelsea Reach Tower, Worlds End Estate, Chelsea London SW10 0EG (GB)
(72) Inventor: Matsumoto, Kaizen Robert, 18 Chelsea Reach Tower, Worlds End Estate, Chelsea London SW10 0EG (GB)
(74) Representative: Stephen, Robert John
(86) International application number: PCT/GB2005/004312
(87) International publication number: WO 2006/059063

(56) References cited:
- EP-A- 0 279 379
- WO-A-93/09762
- GB-A- 2 379 608
- US-A- 4 815 446
- US-A- 5 531 219
- SEKINS, K. M. ET AL: "Feasibility of lung cancer hyperthermia using breathable perfluorochemical (PFC) liquids. Part II: Ultrasound hyperthermia" INTERNATIONAL JOURNAL OF HYPERTHERMIA , 20(3), 278-299 CODEN: IJHYEQ; ISSN: 0265-6736, 2004, XP008068288
- DATABASE WPI Section Ch, Week 199608 Derwent Publications Ltd., London, GB; Class B05, AN 1996-077327 XP002396455 -& WO 96/00568 A1 (BELOV I V) 11 January 1996 (1996-01-11)
- DATABASE WPI Section Ch, Week 198623 Derwent Publications Ltd., London, GB; Class B05, AN 1986-147746 XP002396456 & JP 61 083121 A (GREEN CROSS CORP) 26 April 1986 (1986-04-26)
- DATABASE WPI Section Ch, Week 198835 Derwent Publications Ltd., London, GB; Class A96, AN 1988-246697 XP002396457 & JP 63 179822 A (YAGITA A) 23 July 1988 (1988-07-23)
- ROCKWELL S ET AL: "MODULATION OF TUMOR OXYGENATION AND RADIOSENSITIVITY BY PERFLUOROOCTYLBROMIDE EMULSION" RADIOTHERAPY AND ONCOLOGY, vol. 22, no. 2x, 1991, pages 92-98, XP008068235 ISSN: 0167-8140
- CAMPBELL B H ET AL: "Treatment-related toxicities with Fluosol-DA 20% infusion during radiation in advanced head and neck malignancies" LARYNGOSCOPE 1990 UNITED STATES, vol. 100, no. 3, 1990, pages 237-239, XP008068236 ISSN: 0023-852X
- GOODMAN R L ET AL: "PERFLUOROCARBON EMULSIONS IN CANCER THERAPY PRELIMINARY OBSERVATIONS ON PRESENTLY AVAILABLE FORMULATIONS" INTERNATIONAL JOURNAL OF RADIATION ONCOLOGY, BIOLOGY, PHYSICS, vol. 10, no. 8, 1984, pages 1421-1424, XP008068234 & SYMPOSIUM ON CHEMICAL MODIFIERS OF CANCER TREATMENT, PART 1, BANFF, CANADA, NOV. 27-DEC. 1, 1983. IN ISSN: 0360-3016

## Description

The invention relates to the use of perfluorocarbon liquids, as carcinogen solvents, in medicaments for the prophylactic removal of carcinogens from noncancerous cells, for reducing the risk of cancerous cell transformation.

### Background to the invention

Carcinogens are known to accumulate within the lipophillic environment of the cellular membranes of cells. These accumulated carcinogens represent a carcinogenic reservoir that increases the risk of the cell becoming cancerous. The use of a carcinogen solvent can decrease the risk of cancerous transformation of the cell [1].

Increasing lipophillicity increases the carcinogenic potential of the carcinogen, as the carcinogen is present in the cell for a longer period and is not removed [2].

Carcinogens accumulate within the cellular membranes of cells due to a passive partitioning mechanism, the carcinogens are large organic molecules and therefore lipophillic, and the cellular membrane is phospholipid-based and therefore also lipophillic [3].

This principle of passive partitioning was used to explain the accumulation and localisation of lipophillic carcinogens within cellular membranes [4].

This passive partitioning mechanism is dependent on the relative volumes and lipophillicity of the external solvent and the cellular membranes [4].

The principle of passive partitioning also explained how lipophillic chemicals could be extracted from cellular membranes using lipoproteins in aqueous solution. Lipoproteins are proteins with lipid moieties; the lipid moieties create a lipophillic environment in which lipophillic carcinogens can dissolve, and thus the lipoproteins act as carcinogen solvents [5].

Exposing cellular membranes with intra-cellularly dissolved carcinogens to these lipoprotein solutions alters the relationship between the relative volumes and lipophillicity of the intra-cellular environment and the extra-cellular environment. If the extra-cellular environment, i.e. the lipoprotein solutions, is sufficiently lipophillic, the intra-cellularly dissolved carcinogens will passively partition out of the cells and into the extracellular lipoprotein solution, acting as a carcinogen solvent.

This extraction process was first and uniquely demonstrated in 1981 by Remsen and Shireman [5].

The experiment demonstrated that benzo(a)pyrene, a common environmental carcinogen, could be extracted with a variety of lipoprotein solutions at different concentration, it is also the sole experiment demonstrating the removal of internally dissolved carcinogens from cellular membranes using lipoprotein solutions as carcinogen solvents.

### Description of the invention

This invention describes how the class of chemicals known as perfluorocarbon liquids can be used in a similar manner to lipoproteins, ie as carcinogen solvents, in the removal of carcinogens dissolved within the cells of the body. The removal of carcinogens from cells thereby reduces the risk of cancerous cell transformation. No other extraction medium, ie carcinogen solvent, apart from aqueous- lipoprotein solutions and perfluorocarbon liquids is known to the art or has been reported in the known art as a potential extra-cellular carcinogen solvent for these lipophillic carcinogens.

The invention relates to the use of perfluorocarbon liquids as carcinogen solvents in the manufacture of a medicament for use in vivo to decrease the risk of cancerous transformation of a cell, wherein the cell is exposed to the solvent for at least an hour.

Perfluorocarbon liquids are a class of chemicals defined as being any hydrocarbon where all hydrogen atoms have been substituted with an atom from the halide family such as fluorine, chlorine or bromine.

The presence of the large halide atoms makes all perfluorocarbon liquids inherently lipophillic and thus capable of dissolving out lipophillic carcinogens from cellular membranes, and being used in this invention, as carcinogen solvents.

Various perfluorocarbon are known within the literature, and are envisaged as appropriate for this invention, as all perfluorocarbons are inherently lipophillic.

The fundamental properties required for a perfluorocarbon to be used in this invention as a carcinogen solvent, is that the perfluorocarbon should be liquid and should be lipophillic.

### Examples.

Two perfluorocarbon liquids, perfluorooctylbromide and perfluorodecalin, were tested for their ability to remove a model carcinogen, benzo(a)pyrene, from human fibroplasts. The chemical used, benzo(a)pyrene is an environmental carcinogen, produced in the incomplete combustion of organic materials. It is a lipophillic carcinogen, generally regarded as a good model for the behaviour of other such lipophillic carcinogens [5].

The cells were exposed to radiolabeled benzo(a)pyrene, after exposure the cells were washed with buffer. The cells were then exposed to the perfluorocarbon liquid. Aliquots of the perfluorocarbon liquid were removed at intervals and the radioactivity measured with a scintillation counter. After 120 minutes the cells were tested for membrane integrity with a vital dye, and the membrane and cells were found to be viable, for further reference on the biological effect of perfluorocarbons on phospholipid bilayers see Lack of effect of perfluorooctylbromide on phoshpolipid bilayers, [6]

Following this, the cells were dissolved in acid, and the radioactivity remaining in the cells was measured in a scintillation counter. These measurements were then used to calculate the relative carcinogen extraction in percentage terms against time of exposure to the perfluorocarbon liquid.

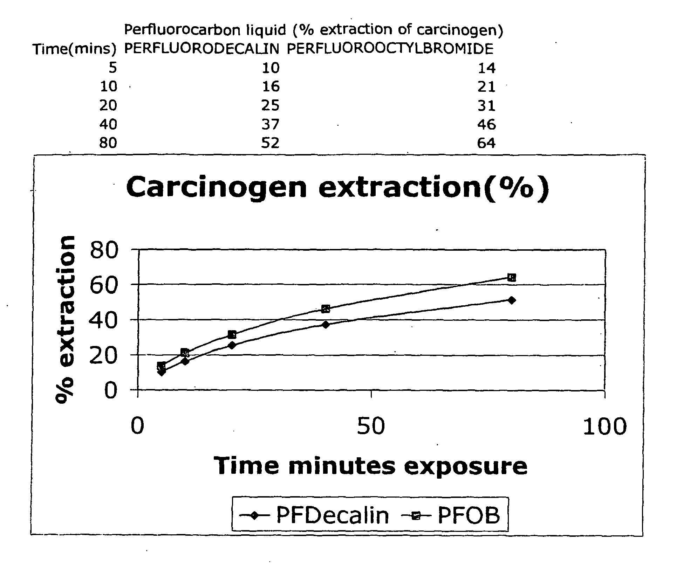

The perfluorocarbons were able to remove between 50% and 65% of the benzo(a)pyrene in an 80 minute exposure, see drawing 1/1, compared to lipoprotein solution which removed 43% and growth medium which removed 5%, data not shown.

Although the cell type used for this in vitro extraction was a human pulmonary cell type, it is envisaged that the invention will work on any cell type, from any organism, as all cellular membranes have similar lipophillic properties. The membranes of cells are necessarily lipophillic as they are made of phospholipids, where the lipid moieties form the intra-membrane space.

This invention thus describes that the perfluorocarbon liquids, perfluorooctylbromide, Perfluorodecalin and perfluorocarbon liquids in general can be used as biologically compatible carcinogen solvents in a formulation of a medicament for the removal of carcinogens from cells. The cells to be treated need to be exposed to the perfluorocarbon liquid medicament for at least an hour, for the carcinogens to equilibrate between the intra-cellular environment and the extra-cellular carcinogen solvent, in the case of this invention a biologically inert perfluorocarbon liquid. Following equilibration of the carcinogen between the extracellular perfluorocarbon carcinogen solvent and the intra-cellular environment, the perfluorocarbon liquid can be removed, and thus effect the removal of carcinogens from cells.

Criteria for choosing suitable perfluorocarbon liquids for use in this invention.

### Lipophillicity

Perfluorocarbons deemed suitable for this invention would need to be lipophillic and generally insoluble in water. This is true for all perfluorocarbons, the substitution of hydrogen atoms by halide atoms creates the high lipophillicity of perfluorocarbon liquids.

### Boiling point

The perfluorocarbon used would need to be liquid at normal body temperature, so that the cells can be exposed to the liquid form of the perfluorocarbon without inducing a temperature shock. Also, a perfluorocarbon gas cannot act as a carcinogen solvent.

### Biological compatibility

The perfluorocarbon used would also need to be non-toxic to the cell; this is true for nearly all perfluorocarbon liquids, and true for the used examples, perfluorooctylbromide and perfluorodecalin.

Known perfluorocarbon liquids suitable for use in the human body and envisaged as proven/obvious candidates for use in this invention include;
Perfluorodecalin (F2 chemicals limited),
Perfluorooctylbromide (Exfluor research corporation),
FC-84 (Fluorinert™ 3M corporation),
FC-72 (Fluorinert™ 3M corporation),
FC-75 (Fluorinert™ 3M corporation),
RM-82 (Perflutel™ Miteni corporation),
RM-lOl (Perflutel™ Miteni corporation).

This is list is not exhaustive and various other perfluorocarbons liquids have been tested for human medical use and can be deemed to be suitable for this invention, if they meet the lipophillicity criteria. Those skilled in the art of perfluorocarbon medicaments will appreciate that since all perfluorocarbon liquids are inherently lipophillic practically any existing perfluorocarbon liquid can be used as a carcinogen solvent in the removal of intra-cellularly dissolved carcinogens.

### Duration of exposure

As the invention describes, the cells in need of carcinogen removal would need to be exposed for an hour at least, preferably eighty to a hundred minutes, for the carcinogens to equilibrate between the intracellular environment and the perfluorocarbon medicament.

Following exposure of the cells by the perfluorocarbon medicament, the perfluorocarbon medicament should be removed, and thereby permanently remove the carcinogens from the cells. If the perfluorocarbon is left to evaporate the carcinogens will re-enter the cells, as the extra-cellular environment can longer dissolve them.

### Background on perfluorocarbons

Perfluorocarbon liquids are known medicaments with various properties that make them suitable for use in the human body.

Perfluorocarbons are odourless, colourless and biologically inert. Their low boiling temperatures and high volatility mean that they do not accumulate in the body. Their extremely low toxicity means that any residual perfluorocarbon does not detrimentally affect the body, and their high chemical stability means that they do not spontaneously break down into toxic products. They are biologically inert, and do not interfere in biological pathways.

They have various other properties, which make them suitable for various therapies. Several uses for perfluorocarbon have been found since their first use as liquid ventilating mediums, these include uses such as liquid ventilation, blood substitutes, ultrasound-heating agent, pulmonary lavage agents, eye surgery, drug delivery agents, surfactant substitutes, radiograph contrast agents, and ultrasound contrast agents. Although the use of perfluorocarbon liquids as pulmonary lavage agents is part of the state-of-the-art, this inventions describes the use of perfluorocarbon based medicaments in the removal of carcinogens already dissolved into cells, not congestive materials which can not dissolve into the cells.

Furthermore, in the known use of perfluorocarbon liquids as lung lavage agents, the perfluorocarbon is only briefly exposed to the pulmonary tissue to remove congestive materials, which is insoluble in the perfluorocarbon liquid. The congestive material floats on top of the highly dense perfluorocarbon liquid, and does not dissolve into the perfluorocarbon liquid.

The use of perfluorocarbon lung lavage is for critical care use, where the patients' breathing is imminently threatened by a physical blockage in the airways. Perfluorocarbon lung lavage is performed in a very transient manner in which the perfluorocarbon liquid is used only to displace the airway blocking material; exposure of the cells to the perfluorocarbon liquid is measured in seconds and minutes. This invention does not relate to blockages of the airways, or as a critical care use of perfluorocarbon liquids. Furthermore the intra-cellularly dissolved carcinogens do not block the airways of the lung.

### Preferred embodiments

### In vivo Detoxification

In its preferred embodiment the invention would constitute a pure perfluorocarbon liquid medicament, either perfluorodecalin, perfluorooctylbromide or a lipophillic perfluorocarbon liquid chosen according to the stated criteria, used through exposing the cells in need of detoxification to the perfluorocarbon medicament for a period of at least one hour, preferably eighty to a hundred minutes, for maximum removal of carcinogens to occur. Following exposure the perfluorocarbon medicament should be removed from the organ as a liquid, and not left to evaporate. The perfluorocarbon medicament can be applied directly to the cells in need of carcinogen removal. The methods and precautions needed for use of perfluorocarbon medicaments in the various organs of the body are known to those skilled in the art. Various organs of the body are known to have higher carcinogen concentrations, and these higher carcinogen concentrations can lead to cancerous cells and tumours. The perfluorocarbon used in the medicament could be any of several known perfluorocarbons with high lipophillicity. The perfluorocarbon would need to be otherwise biologically inert, and liquid at the temperature of treatment. In the case of human use, the perfluorocarbon would need to be liquid at body temperature.

Depending on the organ or tissue to be exposed, other properties of the perfluoroparbon medicament would need to be considered. For use in the lung tissue, or internal organs of the body, the perfluorocarbon liquid would need to be able to dissolve high amounts of gases. Selection criteria for a perfluorocarbon liquid capable of being used in the lung tissue or other internal organs of the body are obvious and known to the art.

For a review of potentially suitable perfluorocarbons for use in the body see Biro.p, Blais P: Perfluorocarbon blood substitutes, CRC critical reviews in oncology/haematology, Vol 6, No.4, p 311-374, 1987.

### Reference Embodiment: Diagnostic

As the perfluorocarbon liquid medicament is retrieved it can be analysed to quantify the presence of the removed carcinogens. Such quantification could be used as a diagnostic tool to quantify the life-long exposure to the various carcinogens, and the current risk associated with those doses.

### Reference Embodiment: In-vitro detoxification

It is envisaged that the perfluorocarbon liquid medicament could be used in a laboratory setting to remove carcinogens from cells in culture.

Such a use could be useful in determining effective dose to the cells of the carcinogen, or determining metabolised proportion of the carcinogens, and other such uses.

For in vitro use, the perfluorocarbon liquid need only be lipophillic and liquid at the temperature the experiment is conducted.

### Reference

[1] High-density lipoproteins decrease both binding of a polynuclear aromatic hydrocarbon carcinogen to DNA and carcinogen-initiated cell transformation. Mutation research.1983, Nov :11 1(3): 429-439. D Busbee, W Benedict.
[2] Correlation of the octanol/water partition coefficient with clearance half-times of intratracheally instilled aromatic hydrocarbons in rats. Toxicology.1985, sep;36(4)p 285-295. J.Bond, S.Baker, W.Bechtold.
[3] Mechanism and rate of permeation of cells by Polycyclic aromatic Hydrocarbons, The journal of biological chemistry, vol 262, No.6, February, 1987, p2514-2519. A.Plant, R. Knapp, L. Smith,
[4] Cellular uptake and intracellular localisation of benzo(a)pyrene by digital fluorescence imaging microscopy, The journal of cell biology, Volume 100, April 1985, p 1295-1308). A Plant, D Benson, L Smith
[5] Removal of benzo(a)pyrene from cells by various components of medium Cancer Letters, 1981 oct:14(l) p41-46 J Remsen, R Shireman:
[6] Lack of effect of perfluorooctylbromide on phospholipid bilayers. Biophysical journal (annual meeting abstracts) Ellena et al, 82(1) pi 57
[7] Perfluorocarbon blood substitutes, CRC critical reviews in oncology/haematology, Vol 6, No.4, p 311-374, 1987. Biro.p, Blais P:

## Claims

1. The use of perfluorocarbon liquids as carcinogen solvents in the manufacture of a medicament for use in vivo to decrease the risk of cancerous transformation of a cell, wherein the cell is exposed to the solvent for at least an hour.

2. The use of claim 1, wherein the perfluorocarbon liquid is selected from:
Perfluorooctylbromide; Perfluorodecalin; FC-84; FC-72; RM-82; FC-75; RM-101.

3. The use of any of the preceding claims, wherein the perfluorocarbon liquid medicament is used on pulmonary cells.

4. The use of any of the preceding claims, wherein the perfluorocarbon liquid medicament is applied to the intestinal tract cells.

5. The use of any of the preceding claims, wherein the perfluorocarbon liquid medicament is used on skin cells.

6. The use of any preceding claim further comprising the step of analyzing said perfluorocarbon liquid for the presence of carcinogens following removal of said perfluorocarbon liquid.

## Patentansprüche

1. Verwendung von Perfluorkohlenwasserstoff-Flüssigkeiten als Karzinogen-Lösungsmittel bei der Herstellung eines Arzneimittels zur Verwendung in vivo zur Verringerung des Risikos von kanzeröser Transformation einer Zelle, wobei das Lösungsmittel mindestens eine Stunde lang auf die Zelle einwirken gelassen wird.

2. Verwendung nach Anspruch 1, wobei die Perfluorkohlenwasserstoff-Flüssigkeit unter Perfluoroctylbromid; Perfluordecalin; FC-84; FC-72; RM-82; FC-75 und RM-101 ausgewählt ist.

3. Verwendung nach einem der vorhergehenden Ansprüche, wobei das Perfluorkohlenwasserstoff-Flüssigkeit enthaltende Arzneimittel an Lungenzellen verwendet wird.

4. Verwendung nach einem der vorhergehenden Ansprüche, wobei das Perfluorkohlenwasserstoff-Flüssigkeit enthaltende Arzneimittel auf die Darmtraktzellen aufgebracht wird.

5. Verwendung nach einem der vorhergehenden Ansprüche, wobei das Perfluorkohlenwasserstoff-Flüssigkeit enthaltende Arzneimittel an Hautzellen verwendet wird.

6. Verwendung nach einem der vorhergehenden Ansprüche, ferner umfassend den Schritt des Analysierens der Perfluorkohlenwasserstoff-Flüssigkeit auf die Gegenwart von Karzinogenen mit nachfolgender Entfernung der Perfluorkohlenwasserstoff-Flüssigkeit.

## Revendications

1. Utilisation de liquides perfluorocarbonés en tant que solvants carcinogènes dans la fabrication d'un médicament destiné à être utilisé in vivo pour réduire le risque de transformation cancéreuse d'une cellule, la cellule étant exposée au solvant pendant au moins une heure.

2. Utilisation selon la revendication 1, **caractérisée en ce que** le liquide perfluorocarboné est choisi parmi : le bromure de perfluorooctyle ; la perfluorodécaline ; FC-84 ; FC-72 ; RM-82 ; FC-75 ; RM-101.

3. Utilisation selon l'une quelconque des revendications précédentes, **caractérisée en ce que** le médicament liquide perfluorocarboné est utilisé sur les cellules pulmonaires.

4. Utilisation selon l'une quelconque des revendications précédentes, **caractérisée en ce que** le médicament liquide perfluorocarboné est appliqué aux cellules du tractus intestinal.

5. Utilisation selon l'une quelconque des revendications précédentes, **caractérisée en ce que** le médicament liquide perfluorocarboné est utilisé sur les cellules cutanées.

6. Utilisation selon l'une quelconque des revendications précédentes, comprenant en outre l'étape consistant à analyser ledit liquide perfluorocarboné pour la présence de carcinogènes à la suite de l'enlèvement dudit liquide perfluorocarboné.
